# EUROPEAN PATENT APPLICATION

(11) **EP 4 606 388 A1**
(43) Date of publication of application: **27.08.2025**
(21) Application number: 23851703.1
(22) Date of filing: 04.08.2023
(51) Int. Cl.: A61K 47/52, A61K 9/127, A61K 47/69

(54) **METAL-POLYPHENOL COMPLEX PARTICLE, DRUG-LIPID PARTICLE, METHOD FOR PREPARING SAME, AND USE THEREOF**

(30) Priority: 09.08.2022 CN 202210950179; 09.08.2022 CN 202210950180; 09.08.2022 CN 202210958544
(71) Applicant: Hunan Lonstar Biotech Co., Ltd., Changsha, Hunan 410000 (CN); Hunan Fanaplos Biotechnology LLC., Changsha, Hunan 410000 (CN)
(72) Inventor: WANG, Shan, Changsha, Hunan 410000 (CN); HU, Dun, Changsha, Hunan 410000 (CN); SUN, Yiyi, Changsha, Hunan 410000 (CN)
(74) Representative: Stork Bamberger Patentanwälte PartmbB
(86) International application number: PCT/CN2023/111151
(87) International publication number: WO 2024/032482

(57) **Abstract**

The disclosure relates to the technical field of biological medicines, and particularly provides a metal-chelated polyphenol complex nanoparticle, a drug-lipid particle, preparation methods for the same, and the uses thereof. The present disclosure provides a metal-chelated polyphenol complex as a carrier for drugs for stability, delivery and the like, so that it interacts with other carriers to form a metal-chelated polyphenol complex nanoparticle for effective administration of negatively charged drug. High-efficiency systemic drug delivery can be achieved, while toxicity is significantly reduced compared to LNP containing cationic or ionizable lipids, enabling safe and effective treatment of diseases or disorders.

## Description

phosphatidylcholine, distearoyl phosphatidylcholine, or dilinoleoyl phosphatidylcholine. Other compounds that do not contain phosphorus, such as sphingomyelin, the glycosphingolipid families, diacylglycerols, and beta-acyloxylic acid can be referred to amphiphilic lipids. In addition, the amphiphilic lipid described above may be mixed with other lipids including triglyceride and sterols.

The term "diacylglycerol" refers to a compound having 2-fatty acyl chains, wherein both R¹ and R² may independently has between 2 and 30 carbons bonded to the 1- and 2-position of glycerol by ester linkages. The acyl group may be saturated or has varying degrees of unsaturation. The diacylglycerol has the following Formula 60:

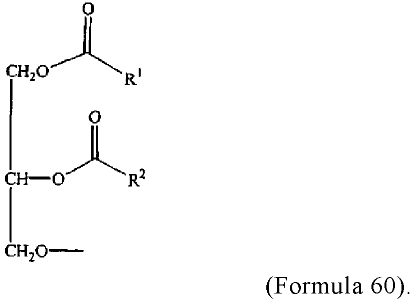

The term "diacylglycerol-coupled polyethylene glycol" i.e., a diacylglycerol-polyethylene glycol conjugate (DAG-PEG conjugate or PEG-DAG conjugate), can be the aggregation-inhibiting conjugated lipid in the present disclosure. In a preferred embodiment, the DAG-PEG conjugate may be dilaurylglycerol (C12)-PEG conjugate, ditetradecylglycerol (C14)-PEG conjugate (DMG), dipalmitoyl glycerol (C16)-PEG conjugate, or distearylglycerol (C18)-PEG conjugate (DSG). Those of skill in the art will readily appreciate that other diacylglycerols can be used for the DAG-PEG conjugate of the present disclosure. Suitable DAG-PEG conjugate for the present disclosure and method of preparation and use thereof them are disclosed in U.S. application No. 10/136,707, published as U.S.P.A 2003/0077829, and PCT patent application No. CA 02/00669, the entire content of which is incorporated herein by reference.

The term "dialkoxypropyl" refers to a compound having 2-alkyl chains, wherein each R¹ and R² may independently has between 2 to 30 carbons. The alkyl group may be saturated or has varying degrees of unsaturation. The dialkoxypropyl group has the following Formula 61:

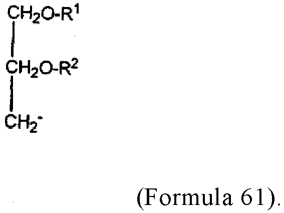

The term "dialkoxypropyl-coupled PEG," i.e., a dialkoxy-propyl conjugate (PEG-DAA conjugate), can be the aggregation-inhibiting conjugated lipid in the present disclosure. In a preferred embodiment, the PEG-DAA conjugate has the following Formula 62:

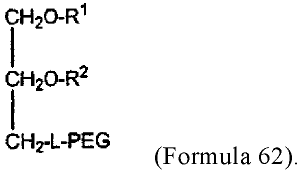

In Formula 62, R¹ and R² may independently be selected and may be a long chain alkyl group having about 10 to 22 carbon atoms. The long chain alkyl group may be saturated or unsaturated. Suitable alkyl groups include, but are not limited to, lauryl (C12), tetradecyl (C14), hexadecyl (C16), octadecyl (C18), and icosyl (C20). In a preferred embodiment, R¹ and R² may be the same, i.e., R¹ and R² may both be tetradecyl (i.e., ditetradecyl), R¹ and R² may both be octadecyl (i.e., dioctadecyl), and the like. In Formula 62, PEG may be a polyethylene glycol having an average molecular weight of about 550 to 10000 Daltons and may optionally be substituted at the terminal hydroxyl position with an alkyl, alkoxy, acyl, or aryl group. In a preferred embodiment, the PEG may have an average molecular weight of about 1000 to 5000 Daltons, more preferably, an average molecular weight of about 1,000 to 3,000 Daltons and even more preferably, an average molecular weight of about 2000 Daltons. PEG may be optionally substituted with alkyl, alkoxy, acyl, or aryl groups. In Formula 62, L is a linker moiety. Any linker moiety suitable for coupling PEG to a dialkoxypropyl backbone may be used. Suitable linker moieties include, but are not limited to, amido (-C (O) NH-), amino (-NR-), carbonyl (-C(O)-), carbonate (O-C(O)O-), carbamate (-NHC (O) O-), urea (-NHC(O)NH-), succinyl (-(O)CCH₂CH₂C(O)-), ether, disulfide, and combinations thereof. Other suitable linker moieties are well known in the art.

Phosphatidylethanolamines, which have various acyl chain groups with different chain lengths and degrees of saturation, can be conjugated to polyethylene glycol to form a bilayer stabilizing component as a particle aggregation-inhibiting conjugated lipid in the present disclosure. These phosphatidylethanolamines are commercially available or can be isolated or synthesized using conventional techniques known to those of skill in the art. Preferably, phosphatidylethanolamines comprise saturated or unsaturated fatty acids and have carbon chain lengths in the range of C10-C20. Phosphatidylethanolamines with mono- or di-unsaturated fatty acids and mixtures of saturated and unsaturated fatty acids can also be used. Suitable phosphatidylethanolamines include, but are not limited to, dimyristoyl phosphatidylethanolamine (DMPE), dipalmitoyl phosphatidylethanolamine (DPPE), dioleoyl phosphatidylethanolamine (DOPE), and distearoyl phosphatidylethanolamine (DSPE).

Ceramides, similar to phosphatidylethanolamines, having various acyl chain groups with different chain lengths and degrees of saturation, can be conjugated to polyethylene glycol to form a bilayer stabilizing component as a particle aggregation-inhibiting conjugated lipid in the present disclosure. It should be appreciated to those of skill in the art that ceramides, in contrast to phosphatidylethanolamine, have only one acyl group, which can be easily varied based on its chain length and degree of saturation. Ceramides suitable for use of the present disclosure are commercially available. In addition, ceramides may be isolated, for example, from eggs and brain using well-known seperation techniques, or synthesized using the methods and techniques disclosed in U.S. Patent No. 5,820,873, which is incorporated herein by reference. Using the synthetic routes set forth in the aforementioned applications, ceramides may be prepared with saturated or unsaturated fatty acids having carbon chain lengths in the range of C2-C31.

The term "ATTA" or "polyamide" refers to, but is not limited to, compounds disclosed in U.S. Patent Nos. 6,320,017 and 6,586,559, which are incorporated herein by reference. These compounds include a compound having the following Formula 63:

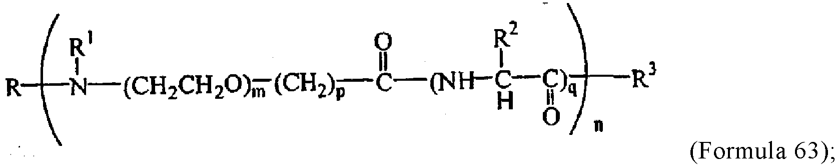

Wherein: R may be selected from the group consisting of hydrogen, alkyl, and acyl; R¹ may be selected from the group consisting of hydrogen and alkyl; or optionally, R and R¹ and the nitrogen atom to which they are bound may form an azido moiety; R² may

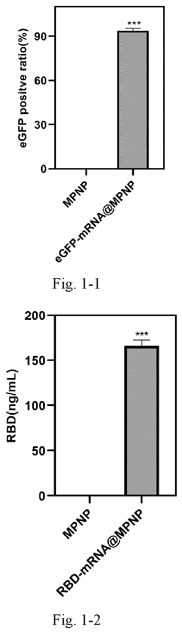

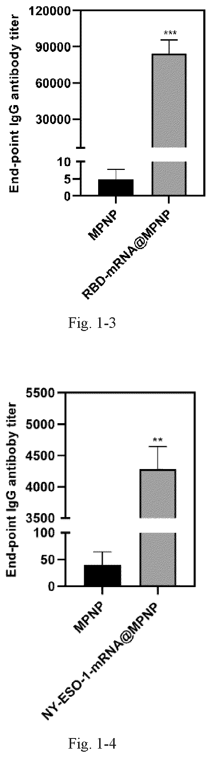

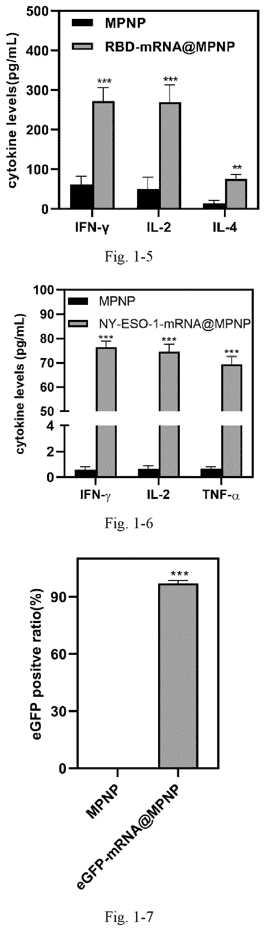

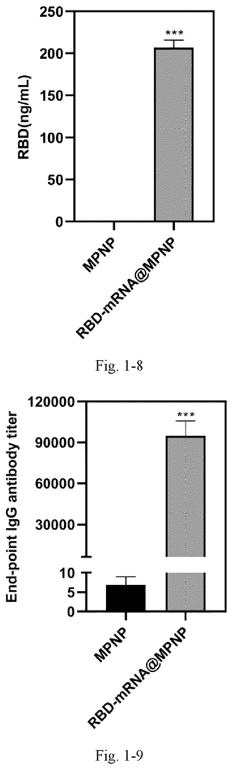

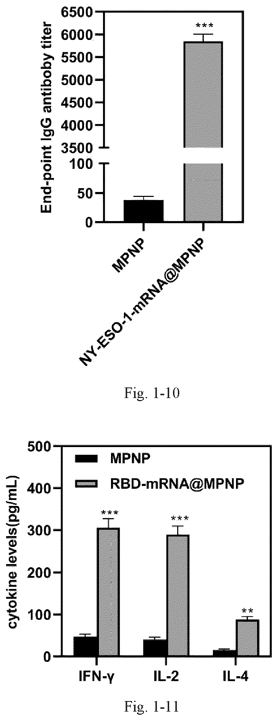

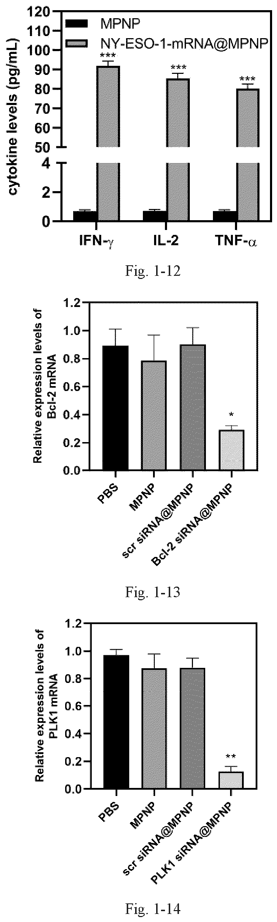

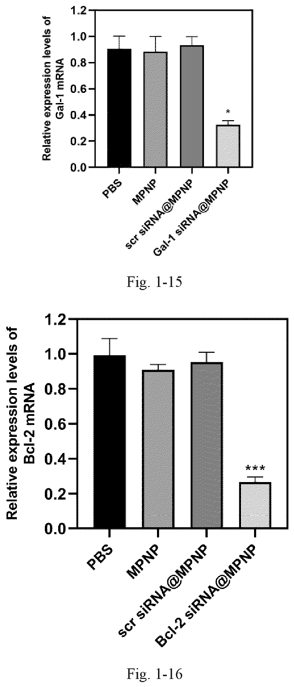

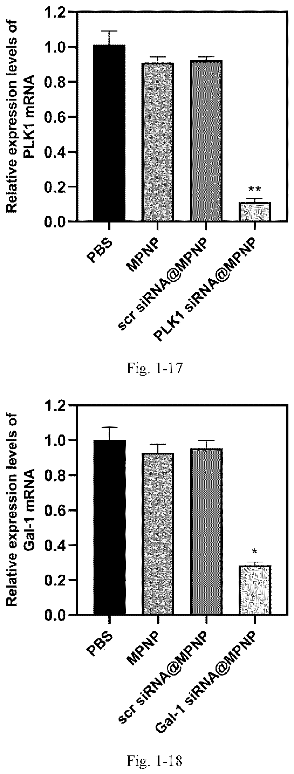

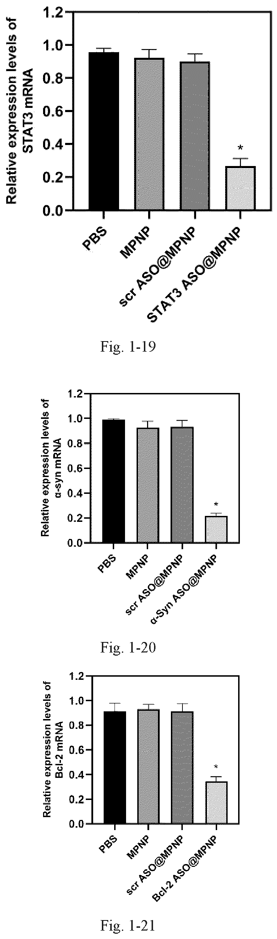

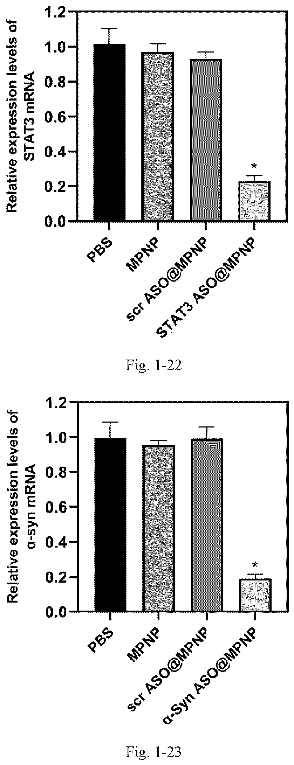

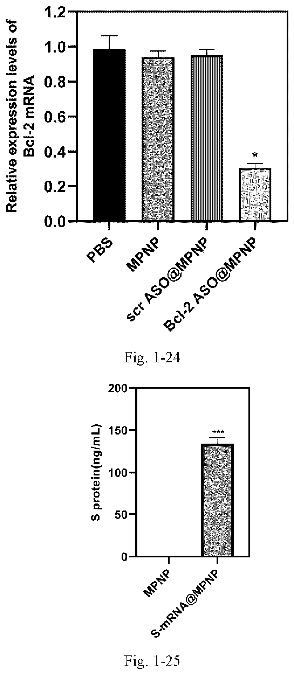

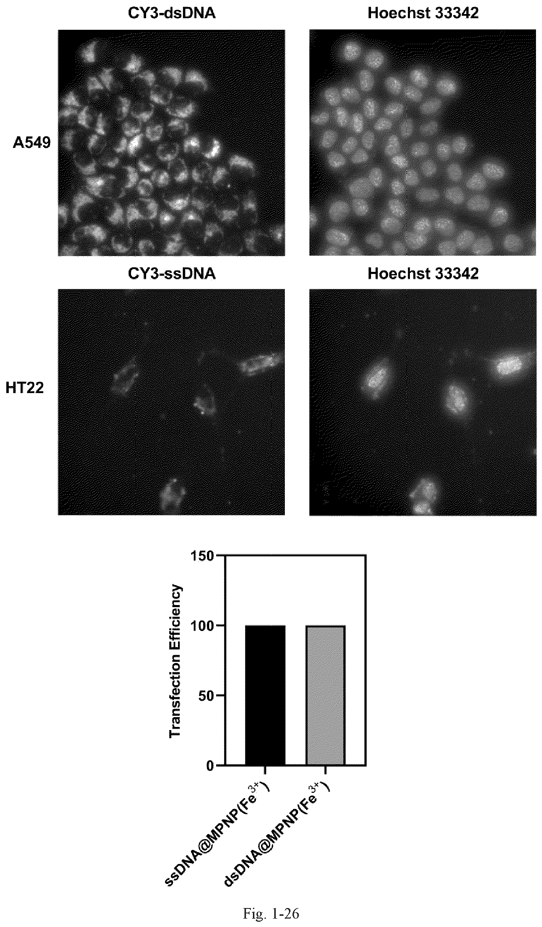

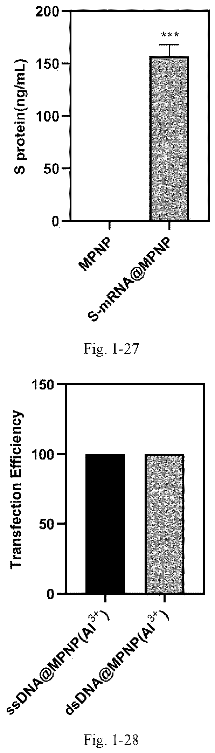

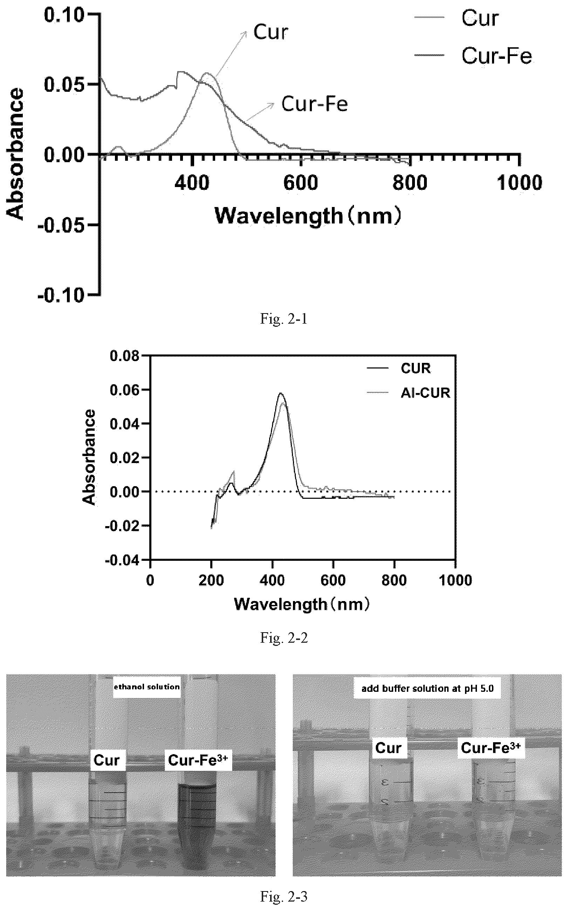

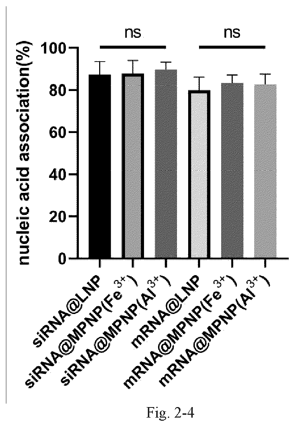

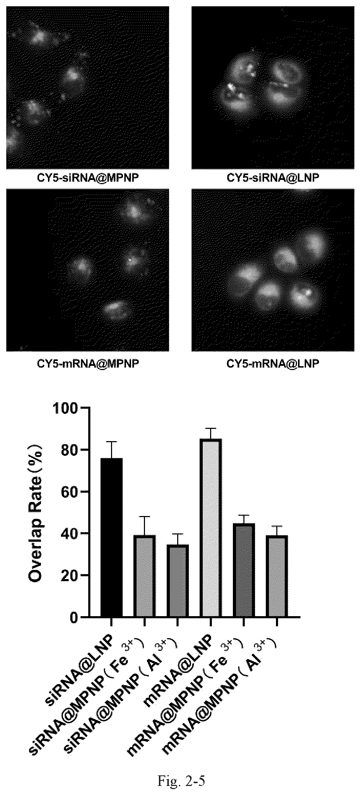

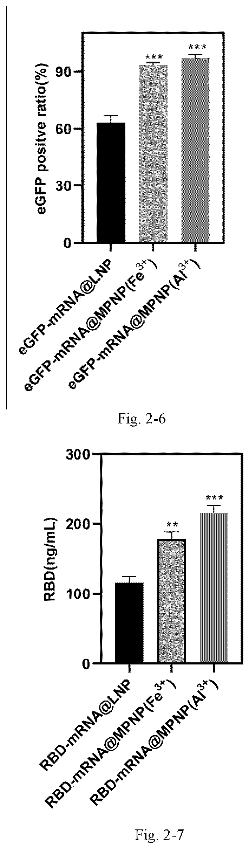

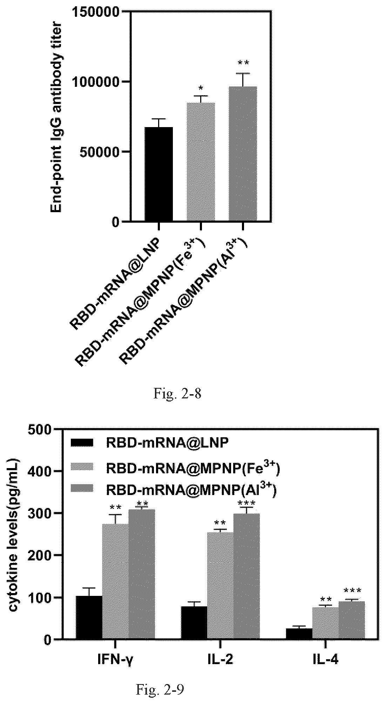

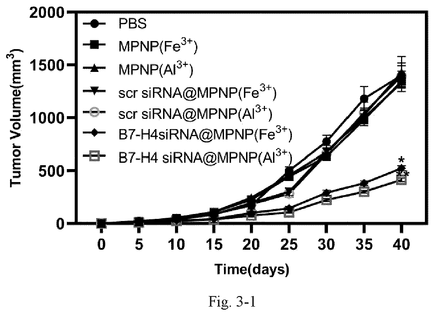

## Claims

1. Use of a metal-chelated polyphenol complex in a nucleic acid delivery system, wherein the metal-chelated polyphenol complex is formed by reaction of a polyphenol molecular moiety and a metal ion moiety, the polyphenol molecular moiety and the metal ion moiety being linked by a coordination bond.

2. The use of claim 1, wherein the polyphenol molecular moiety is selected from the group consisting of curcumin, quercetin, kaempferol, rutin, hesperetin, naringenin, eriodictyol, luteolin, apigenin, toxifolin, phlorotannin, flavanol polyphenol, catechin, ellagic acid, gallic acid, digallic acid, propyl gallate, epigallocatechin gallate, galloylglucose, hydroxy-hydroquinone, morin, epicatechin gallate, catechin gallate, gallocatechin gallate, and derivatives thereof, and combinations thereof.

3. The use of claim 2, wherein the polyphenolic molecular moiety is selected from the group consisting of curcumin (Formula 1), quercetin (Formula 2), kaempferol (Formula 3), rutin (Formula 4), hesperetin (Formula 5), naringenin (Formula 6), eriodictyol (Formula 7), luteolin (Formula 8), apigenin (Formula 9), toxifolin (Formula 10), phlorotannin (Formula 11), flavanol polyphenol (Formula 12), catechin (Formula 13), ellagic acid (Formula 14), gallic acid (Formula 15), digallic acid (Formula 16), propyl gallate (Formula 17), epigallocatechin gallate (Formula 18), galloylglucose (Formula 19), hydroxy-hydroquinone (Formula 20), morin (Formula 21), epicatechin gallate (Formula 22), catechin gallate (Formula 23), gallocatechin gallate (Formula 24), and derivatives thereof, and combinations thereof; 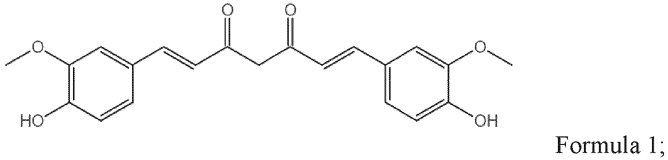 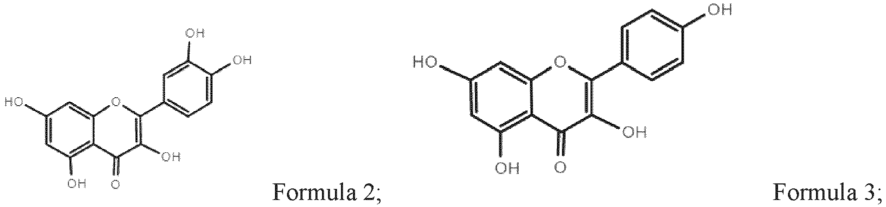 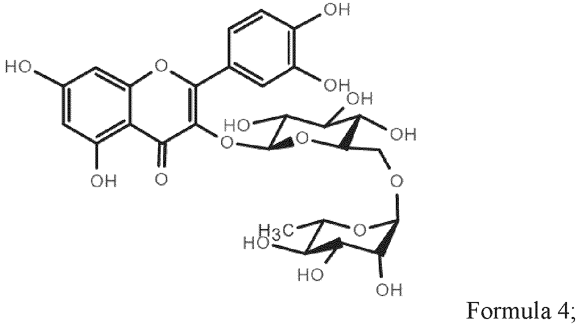 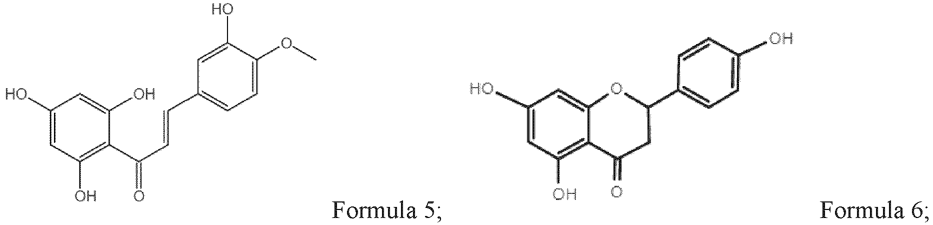 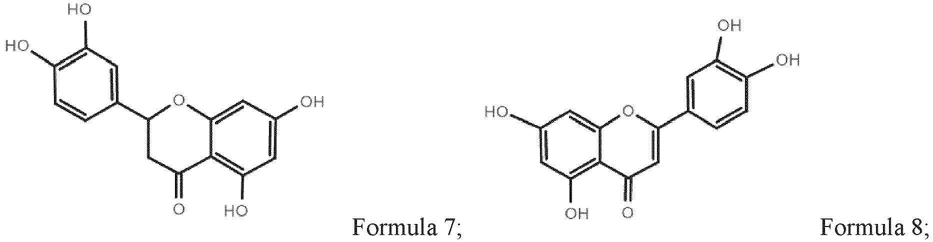 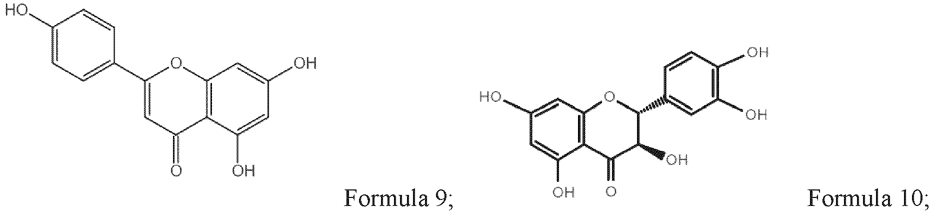 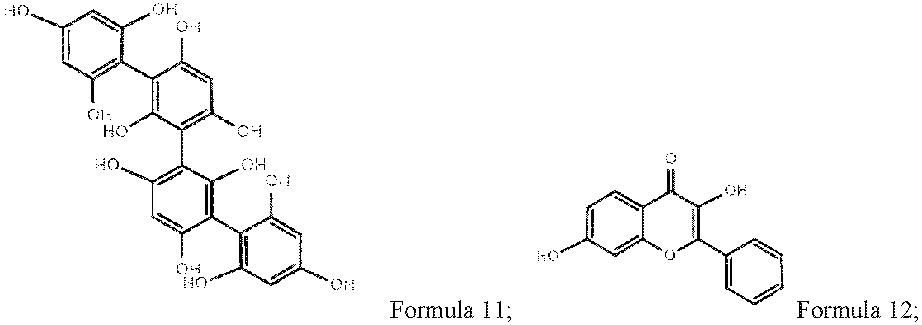 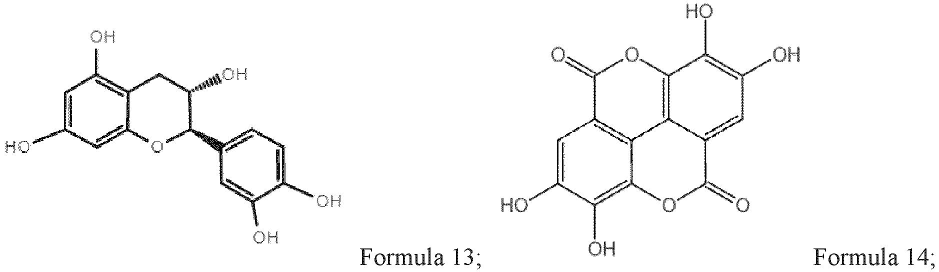 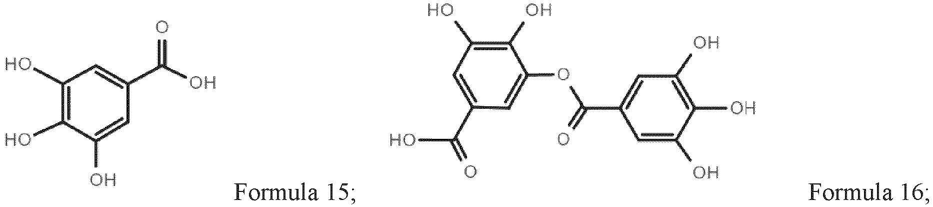 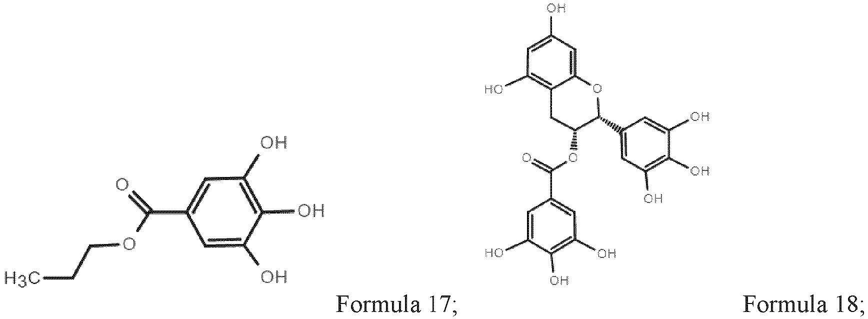 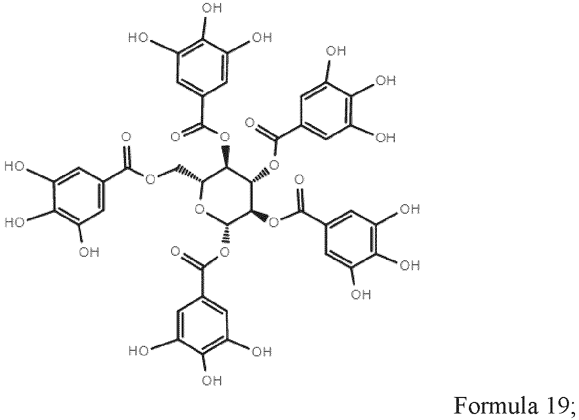 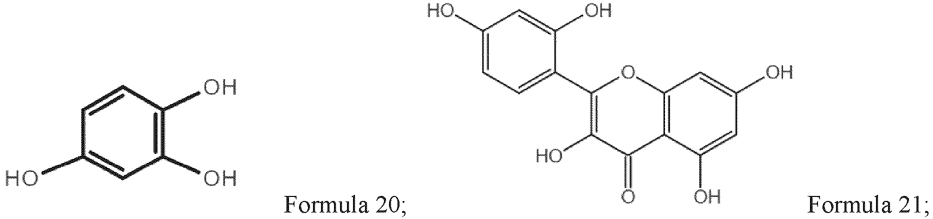 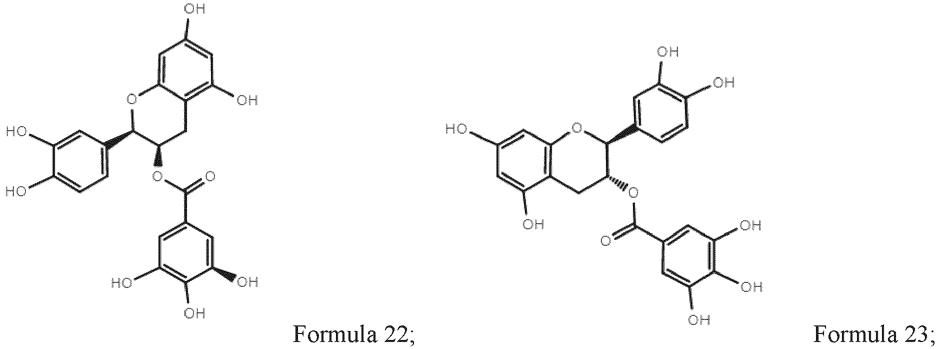 and

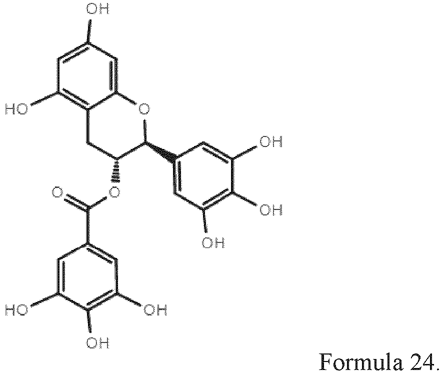

4. The use of claim 3, wherein the polyphenol molecular moiety is selected from the group consisting of curcumin (Formula 1), dihydrocurcumin (Formula 25), hexahydrocurcumin (Formula 26), curcumin sulfate (Formula 27) and bisdemethoxycurcumin (Formula 28) , and combinations thereof; 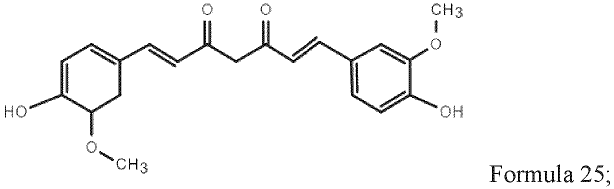 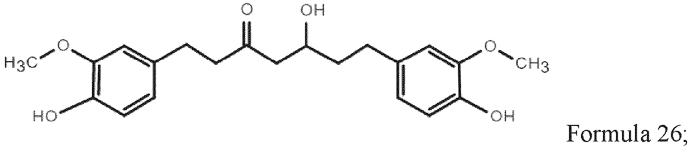 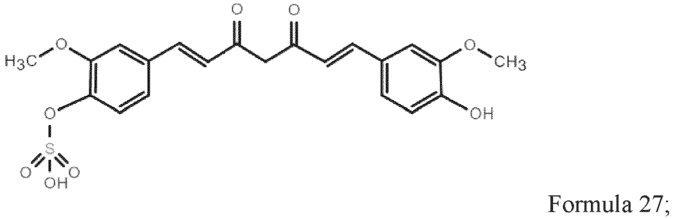 and

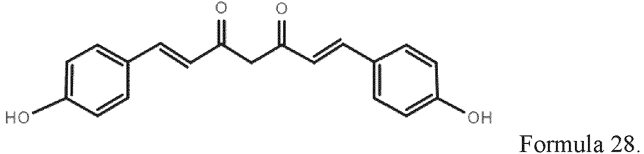

5. The use of claim 3, wherein the polyphenol molecular moiety is selected from the group consisting of curcumin (Formula 1), hesperetin (Formula 5) or catechin (Formula 13), and derivatives thereof, and combinations thereof.

6. The use of claim 5, wherein the polyphenol molecular moiety is selected from curcumin (Formula 1), hesperetin (Formula 5) or catechin (Formula 13).

7. The use of any one of claims 1 to 6, wherein the metal ion moiety is selected from the group consisting of Fe³⁺, Ag⁺, Ba²⁺, Ca²⁺, Cd ²⁺, Cu²⁺, Fe²⁺, Mn²⁺, Mg²⁺, Mo²⁺, Zn²⁺, Pt²⁺, Au²⁺, Al³⁺, Ce³⁺, Co³⁺, Cr³⁺, Eu³⁺, Gd³⁺, Ni³⁺, W³⁺, V³⁺, and Zr³⁺, and combinations thereof.

8. Use of to claim 7, wherein the metal ion moiety is selected from the group consisting of Fe³⁺, Ca²⁺ and Al³⁺, and combinations thereof.

9. Use of claim 8, wherein the metal ion moiety is selected from Fe³⁺, Ca²⁺ and Al³⁺.

10. Use of claim 2 or 7, wherein the metal-chelated polyphenol complex is formed by reaction of the polyphenol molecular moiety selected from curcumin, hesperetin and catechin and the metal ion moiety selected from Fe³⁺, Ca²⁺ and Al³⁺.

11. The use of claim 10, wherein the metal-chelated polyphenol complex is formed by reaction of the polyphenol molecular moiety selected from curcumin (Formula 1), hesperetin (Formula 5) and catechin (Formula 13) and the metal ion moiety selected from Fe³⁺ , Ca²⁺ and Al³⁺.

12. The use of claim 11, wherein the molar ratio of the polyphenol molecular moiety to the metal ion moiety is 1: (0.5 to 2).

13. The use of claim 12, wherein the polyphenol molecular moiety is curcumin (Formula 1) and the metal ion moiety is Fe³⁺.

14. The use of claim 13, wherein the molar ratio of curcumin (Formula 1) to Fe³⁺ is 1: 1.

15. The use of claim 12, wherein the polyphenol molecular moiety is curcumin (Formula 1) and the metal ion moiety is Al³⁺.

16. The use of claim 15, wherein the molar ratio of curcumin (Formula 1) to Al³⁺ is 1: 1.

17. The use of any one of claims 1 to 16, wherein the nucleic acid delivered by the nucleic acid delivery system is selected from the group consisting of mRNA, siRNA, sgRNA, ASO, circRNA, microRNA, DNA, ecDNA and artificial nucleic acid, and combinations thereof.

18. The use of claim 17, wherein the nucleic acid is an mRNA sequence shown in SEQ ID No.1 for encoding eGFP (Enhanced Green Fluorescent Protein), an mRNA sequence shown in SEQ ID No.2 for encoding a receptor binding domain RBD of a novel coronavirus S1 subunit, an mRNA sequence shown in SEQ ID No.3 for encoding NY-ESO-1 (tumor antigen), an siRNA sequence of a Bcl-2 gene (B-cell lymphoma/Leukemia-2) having an antisense strand shown in SEQ ID No.4 and a sense strand shown in SEQ ID No.21, an siRNA sequence of a PLK1 gene (Polo-like Kinase 1) having an antisense strand shown in SEQ ID No.6 and a sense strand showed in SEQ ID No.23, an siRNA sequence of a Gal-1 gene shown in SEQ ID No.8, an ASO sequence of a STAT-3 gene shown in SEQ ID No.10, an ASO sequence of an alpha-syn gene (alpha-synuclein) shown in SEQ ID No.12, an ASO sequence of a Bcl-2 gene shown in SEQ ID No.14, an mRNA sequence shown in SEQ ID No.16 for encoding a wild-type novel coronavirus S protein, a double-stranded DNA sequence having an antisense strand shown in SEQ ID No.17 and a sense strand shown in SEQ ID No.25, a single-stranded DNA shown in SEQ ID No.18, or a siRNA sequence of a B7-H4 gene having a sense strand shown in SEQ ID No.19 and an antisense strand shown in SEQ ID No. 26.

19. The use of any one of claims 1 to 18, wherein the nucleic acid delivery system is used to introduce a nucleic acid into a cell.

20. The use of claim 19, wherein the nucleic acid is used to silence expression of a target sequence in a mammalian subject or to treat a disease or a disorder in a mammal.

21. The use of claim 20, wherein the mammal is a human.

22. The use of claim 20, wherein the disease or the disorder is associated with expression of a gene comprising a target sequence of a drug.

23. The use of claim 22, wherein the disease or the disorder comprises cancer, viral infection, autoimmune disease, diabetes, and Alzheimer's disease.

24. The use of claim 23, wherein the viral infection comprises hepatitis a, hepatitis b, hepatitis c, SARS-Cov-2, HIV, HPV, influenza, smallpox, and syphilis.

25. The use of claim 23, wherein the cancer comprises liver cancer, glioma, melanoma, lung cancer, pancreatic cancer, and breast cancer.

26. Use of claim 1, wherein the nucleic acid delivery system is used for the preparation of a vaccine.

27. Use of claim 26, wherein the vaccine is a novel coronavirus vaccine.

28. A metal-chelated polyphenol complex nanoparticle comprising:
(i) a metal-chelated polyphenol complex formed by a reaction of a polyphenol molecular moiety and a metal ion moiety, the polyphenol molecular moiety and the metal ion moiety being linked by a coordination bond;
(ii) a particle aggregation-inhibiting conjugated lipid, wherein the particle aggregation-inhibiting conjugated lipid is not a cationic lipid or an ionizable lipid; and
(iii) a non-cationic lipid or a non-ionizable lipid other than the particle aggregation-inhibiting conjugated lipid.

29. The metal-chelated polyphenol complex nanoparticle of claim 28, wherein the polyphenol molecular moiety is selected from the group consisting of curcumin, quercetin, kaempferol, rutin, hesperetin, naringenin, eriodictyol, luteolin, apigenin, toxifolin, phlorotannin, flavanol polyphenol, catechin, ellagic acid, gallic acid, digallic acid, propyl gallate, epigallocatechin gallate, galloylglucose, hydroxy-hydroquinone, morin, epicatechin gallate, catechin gallate, gallocatechin gallate, and derivatives thereof, and combinations thereof.

30. The metal-chelated polyphenol complex nanoparticle of claim 29, wherein the polyphenol molecular moiety selected from the group consisting of curcumin (Formula 1), quercetin (Formula 2), kaempferol (Formula 3), rutin (Formula 4), hesperetin (Formula 5), naringenin (Formula 6), eriodictyol (Formula 7), luteolin (Formula 8), apigenin (Formula 9), toxifolin (Formula 10), phlorotannin (Formula 11), flavanol polyphenol (Formula 12), catechin (Formula 13), ellagic acid (Formula 14), gallic acid (Formula 15), digallic acid (Formula 16), propyl gallate (Formula 17), epigallocatechin gallate (Formula 18), galloylglucose (Formula 19), hydroxy-hydroquinone (Formula 20), morin (Formula 21), epicatechin gallate (Formula 22), catechin gallate (Formula 23), gallocatechin gallate (Formula 24), and derivatives thereof, and combinations thereof.

31. The metal-chelated polyphenol complex nanoparticle of claim 30, wherein the polyphenol molecular moiety is selected from the group consisting of curcumin (Formula 1), dihydrocurcumin (Formula 25), hexahydrocurcumin (Formula 26), curcumin sulfate (Formula 27), bisdemethoxycurcumin (Formula 28), and combinations thereof.

32. The metal-chelated polyphenol complex nanoparticle of claim 30, wherein the polyphenol molecular moiety is selected from the group consisting of curcumin (Formula 1), hesperetin (Formula 5), or catechin (Formula 13), and derivatives thereof, and combinations thereof.

33. The metal-chelated polyphenol complex nanoparticle of claim 32, wherein the polyphenol molecular moiety is selected from curcumin (Formula 1), hesperetin (Formula 5), and catechin (Formula 13).

34. The metal-polyphenol composite particle of any of claims 28 to 33 wherein the metal ion moiety is selected from the group consisting of Fe³⁺, Ag⁺, Ba²⁺, Ca²⁺, Cd ²⁺, Cu²⁺, Fe²⁺, Mn²⁺, Mg²⁺, Mo²⁺, Zn²⁺, Pt²⁺, Au²⁺, Al³⁺, Ce³⁺, Co³⁺, Cr³⁺, Eu³⁺, Gd³⁺, Ni³⁺, W³⁺, V³⁺, and Zr³⁺, and combinations thereof.

35. The metal-chelated polyphenol complex nanoparticle of claim 34, wherein the metal ion moiety is selected from the group consisting of Fe³⁺, Ca²⁺ and Al³⁺, and combinations thereof.

36. The metal-chelated polyphenol complex nanoparticle of claim 35, wherein the metal ion moiety is selected from Fe³⁺, Ca²⁺ and Al³⁺.

37. The metal-chelated polyphenol complex nanoparticle of any of claims 28 to 36, wherein the particle aggregation-inhibiting conjugated lipid comprises a PEG-lipid conjugate and/or PEG-DAA.

38. The metal-chelated polyphenol complex nanoparticle of claim 37, wherein the PEG-lipid conjugate is selected from the group consisting of phosphatidylethanolamine-polyethylene glycol 2000 (Formula 47), phosphatidylethanolamine-polyethylene glycol 700 (Formula 48), phosphatidylethanolamine-polyethylene glycol 1000 (Formula 49), phosphatidylethanolamine-polyethylene glycol 5000 (Formula 50), and derivatives thereof, and combinations thereof;
wherein, R1, R2 are each independently:
decanoyl 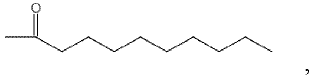
lauroyl 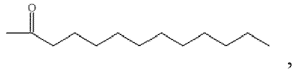
myristoyl 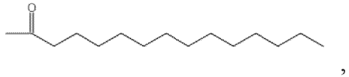
palmitoyl 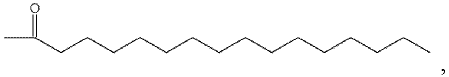
stearyl 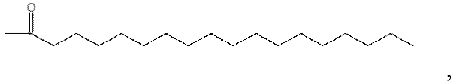
oleoyl 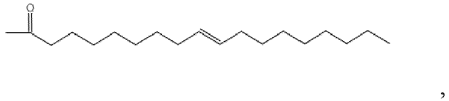
linoleoyl 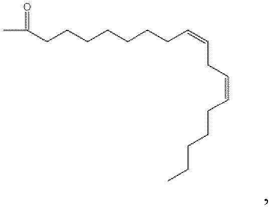
erucoyl 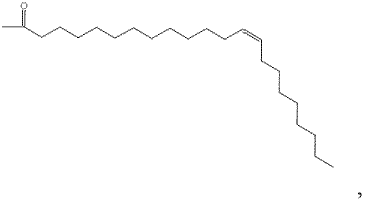
arachidoyl 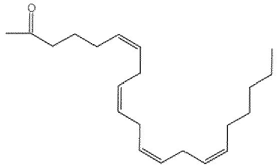 or
phytanoyl 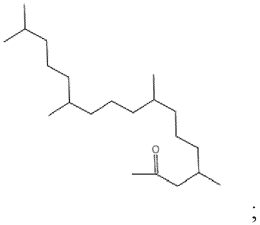 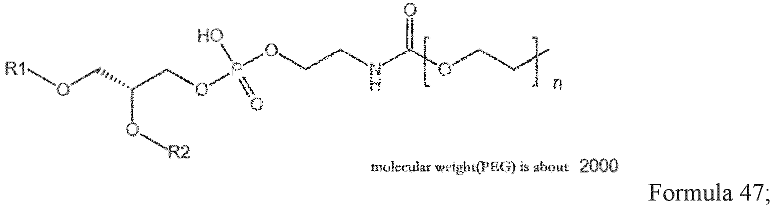 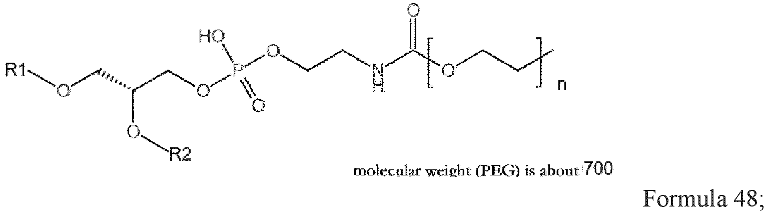 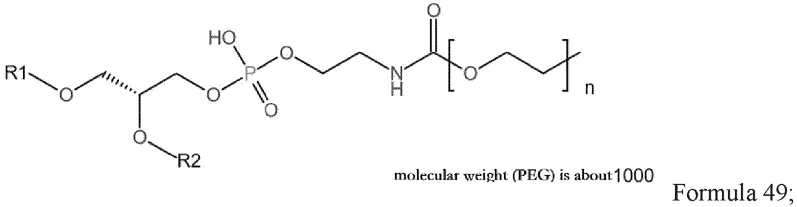 and

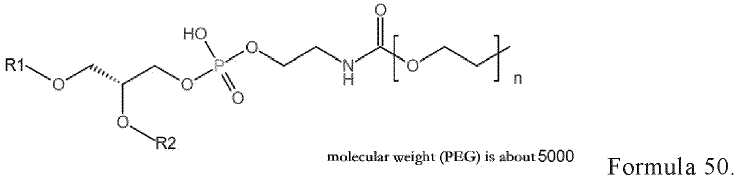

39. The metal-chelated polyphenol complex nanoparticle of claim 38, wherein the PEG-lipid conjugate is selected from the group consisting of DSPE-PEG2000, DSPE-PEG700, DSPE-PEG1000 and DSPE-PEG 5000, and combinations thereof.

40. The metal-chelated polyphenol complex nanoparticle of claim 39, wherein the PEG-lipid conjugate is selected from DSPE-PEG2000 (Formula 58), DSPE-PEG700 (Formula 55), DSPE-PEG1000 (Formula 56) and DSPE-PEG5000 (Formula 57); 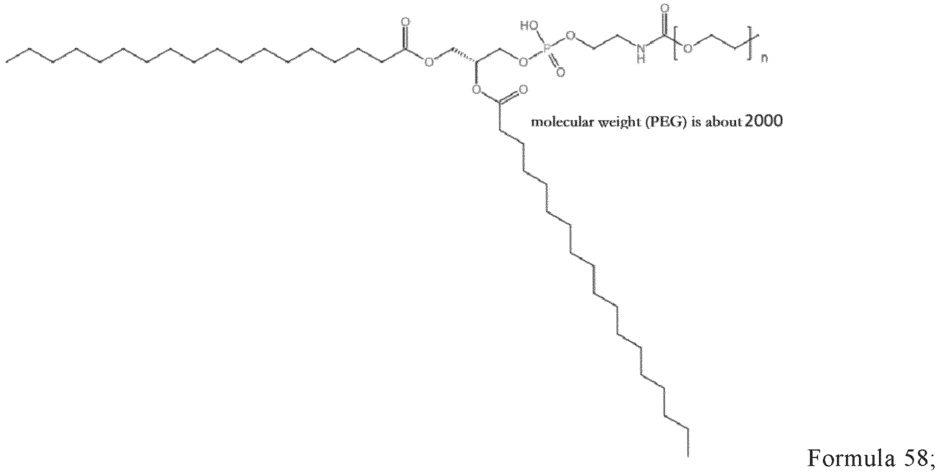 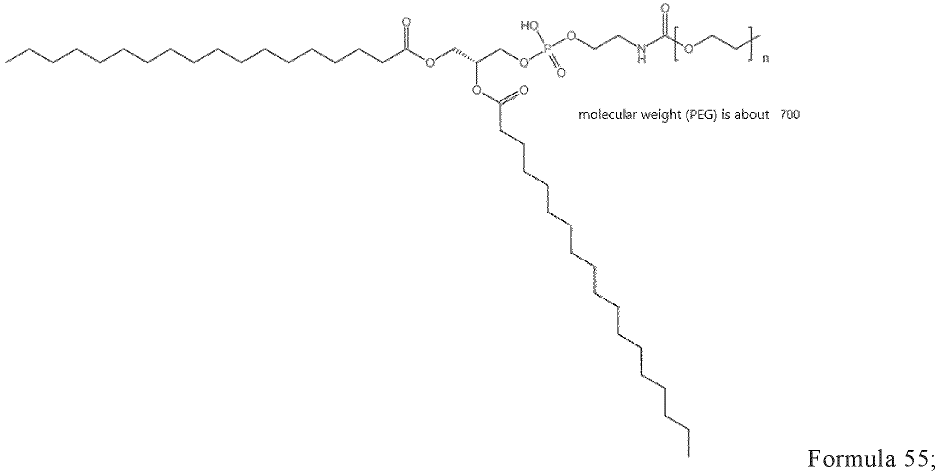 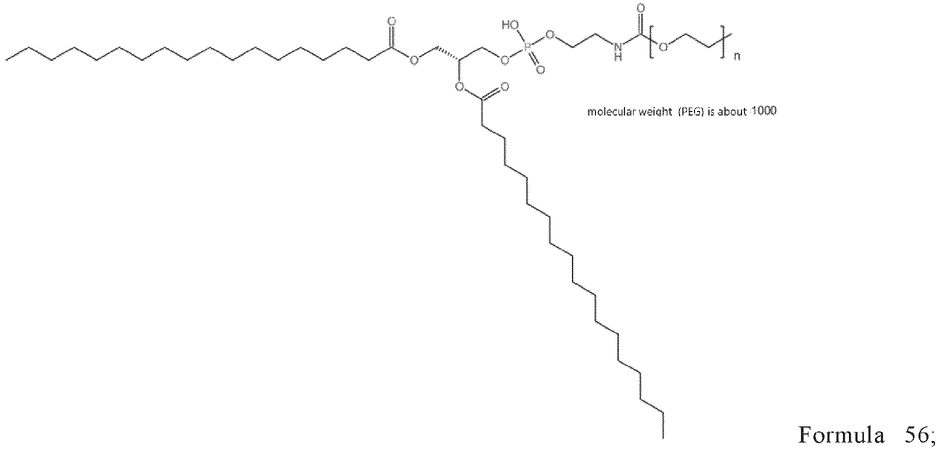 and

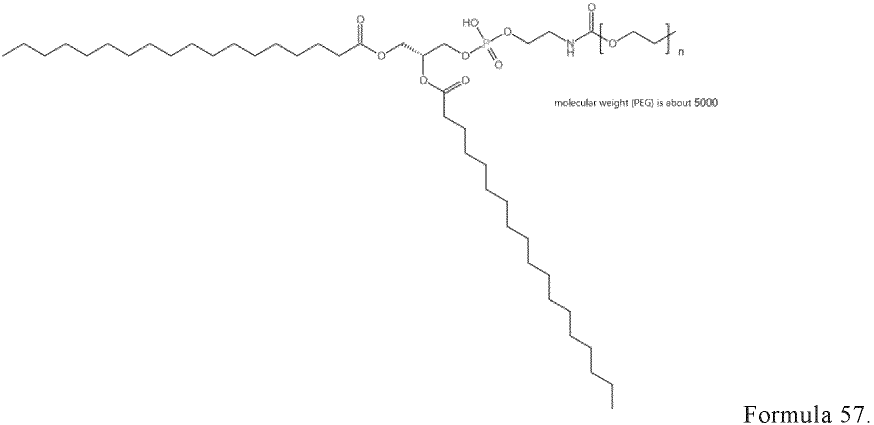

41. The metal-chelated polyphenol complex nanoparticle of any of claims 28 to 40, wherein the non-cationic lipid or non-ionizable lipid in (iii) is selected from the group consisting of lecithin PC, phosphatidyl ethanolamine PE, phosphatidyl serine PS, phosphatidic acid PA, phosphatidyl glycerol PG, ceramide-1-phosphate CP, phosphatidyl inositol PI, phosphatidyl threonine PT, sphingomyelin SM, lysolecithin LPC, lysophosphatidylethanolamine LPE, lysophosphatidylserine LPS, lysophosphatidic acid LPA, lysophosphatidylglycerol LPG, lysophosphatidylinositol LPI, lysophosphatidylthreonine LPT, lysosphingomyelin LSM, sphingosine 1-phosphate S1P, and derivatives thereof, and combinations thereof.

42. The metal-chelated polyphenol complex nanoparticle of claim 41, wherein the non-cationic lipid or non-ionizable lipid in (iii) is selected from the group consisting of lecithin (PC) (Formula 29), phosphatidyl ethanolamine (PE) (Formula 30), phosphatidyl serine (PS) (Formula 31), phosphatidic acid (PA) (Formula 32), phosphatidyl glycerol (PG) (Formula 33), ceramide-1-phosphate (CP) (Formula 34), phosphatidyl inositol (PI) (Formula 35), phosphatidyl threonine (PT) (Formula 36), sphingomyelin (SM) (Formula 37), lysolecithin (LPC) (Formula 38), lysophosphatidylethanolamine (LPE) (Formula 39), lysophosphatidylserine (LPS) (Formula 40), lysophosphatidic acid (LPA) (Formula 41), lysophosphatidylglycerol (LPG) (Formula 42), lysophosphatidylinositol (LPI) (Formula 43), lysophosphatidylthreonine (LPT) (Formula 44), lysosphingomyelin (LSM) (Formula 45), sphingosine-1-phosphate (S1P) (Formula 46), and derivatives thereof, and combinations thereof, wherein, R1 and R2 may each independently be decanoyl, lauroyl, myristoyl, palmitoyl, stearoyl, oleoyl, linoleoyl, erucoyl, arachidoyl or phytanoyl, 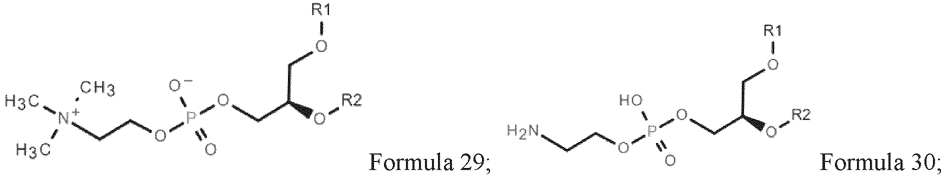 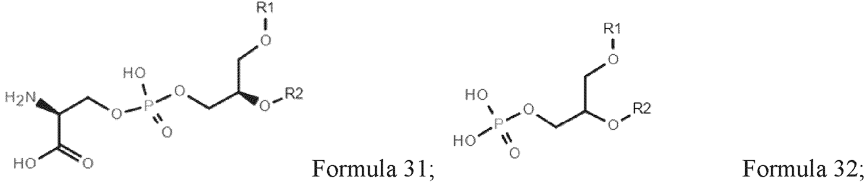 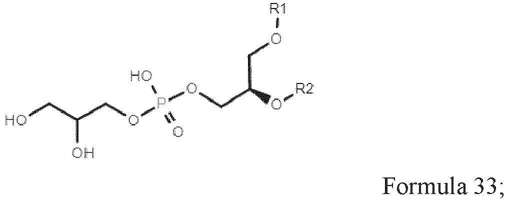 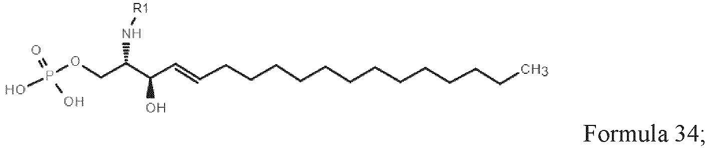 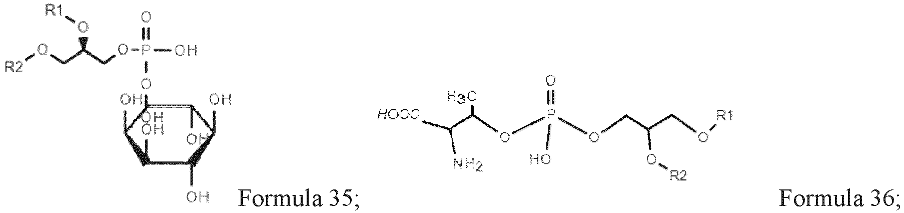 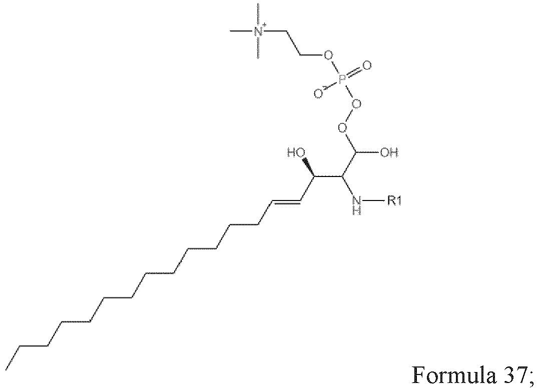 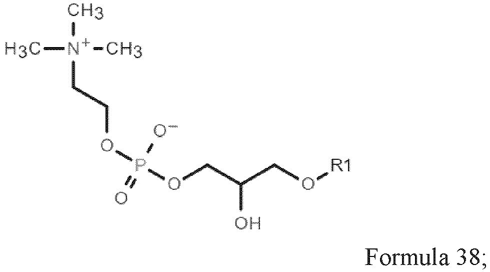 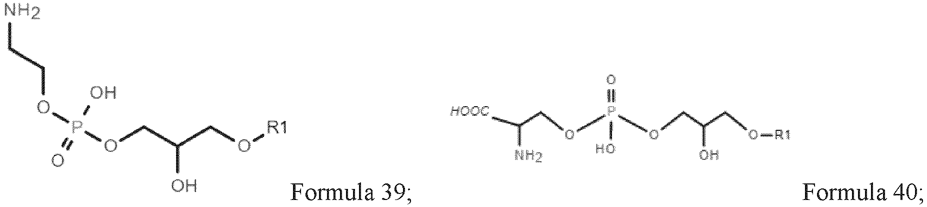 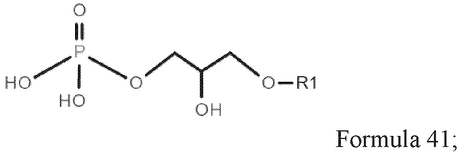 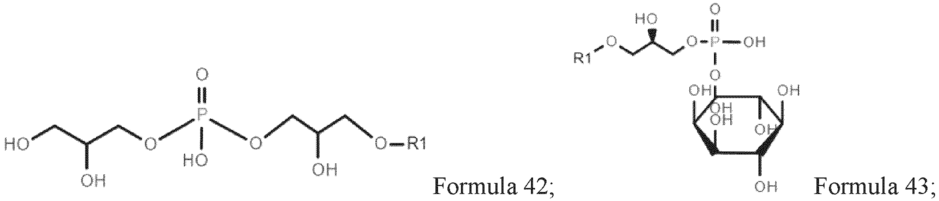 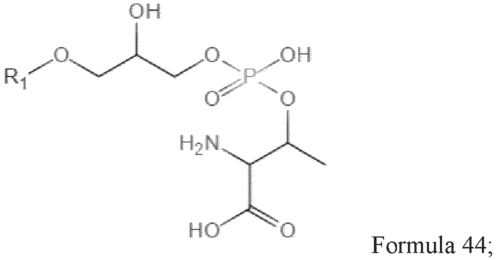 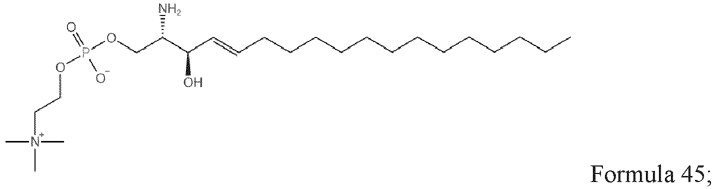 and

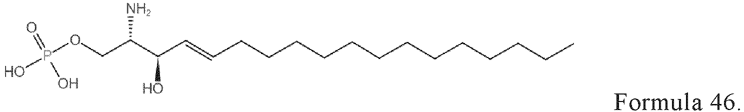

43. The metal-chelated polyphenol complex nanoparticle of claim 41 or 42, wherein the non-cationic lipid or non-ionizable lipid in (iii) further comprises at least one of cholesterol and derivatives thereof.

44. The metal-chelated polyphenol complex nanoparticle of claim 43, wherein the non-cationic lipid or non-ionizable lipid in (iii) comprises cholesterol and one or more selected from the group consisting of DSPC, DSPE, DSPA, and DSPG.

45. The metal-chelated polyphenol complex nanoparticle of claim 44 wherein the non-cationic lipid or non-ionizable lipid in (iii) comprises cholesterol (Formula 59), and one or more selected from the group consisting of DSPC (Formula 51), DSPE (Formula 52), DSPA (Formula 53), and DSPG (Formula 54); 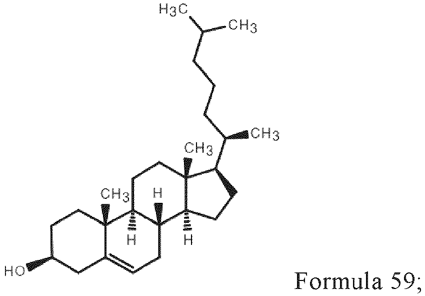 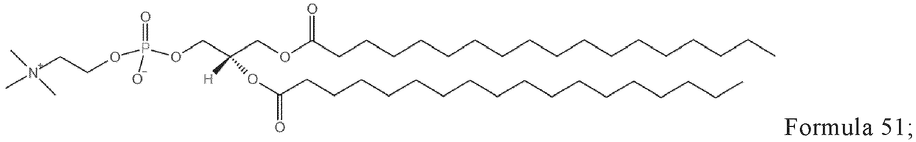 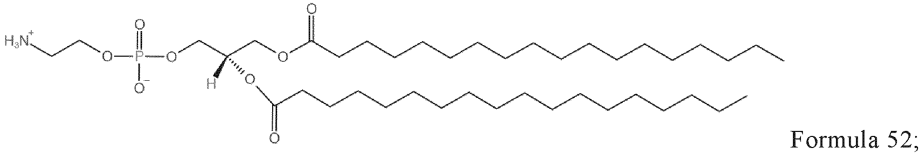 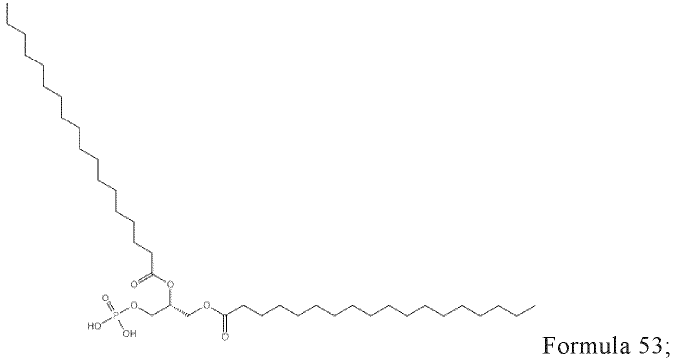 and

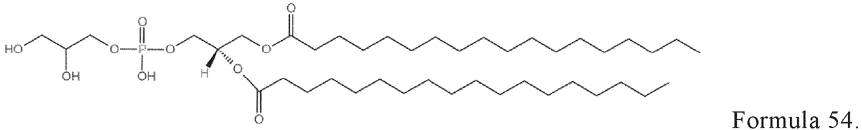

46. The metal-chelated polyphenol complex nanoparticle of claim 45, wherein the non-cationic lipid or non-ionizable lipid in (iii) comprises cholesterol (Formula 59) and DSPC (Formula 51).

47. The metal-chelated polyphenol complex nanoparticle of claim 29 or 34, wherein the metal-chelated polyphenol complex is formed by a reaction of the polyphenol molecular moiety selected from curcumin, hesperetin and catechin and the metal ion moiety selected from Fe³⁺, Ca²⁺ and Al³⁺.

48. The metal-chelated polyphenol complex nanoparticle of claim 47, wherein the metal-chelated polyphenol complex is formed by a reaction of the polyphenol molecular moiety selected from curcumin (Formula 1), hesperetin (Formula 5) and catechin (Formula 13) and the metal ion moiety selected from Fe³⁺, Ca²⁺ and Al³⁺.

49. The metal-chelated polyphenol complex nanoparticle of claim 48 wherein the molar ratio of the polyphenol molecular moiety to the metal ion moiety is 1: (0.5 to 2).

50. The metal-chelated polyphenol complex nanoparticle of claim 49, wherein the polyphenol molecular moiety is curcumin (Formula 1) and the metal ion moiety is Fe³⁺.

51. The metal-chelated polyphenol complex nanoparticle of claim 50, wherein the molar ratio of curcumin (Formula 1) to Fe³⁺ is 1: 1.

52. The metal-chelated polyphenol complex nanoparticle of claim 49, wherein the polyphenol molecular moiety is curcumin (Formula 1) and the metal ion moiety is Al³⁺.

53. The metal-chelated polyphenol complex nanoparticle of claim 52, wherein the molar ratio of curcumin (Formula 1) to Al³⁺ is 1: 1.

54. The metal-chelated polyphenol complex nanoparticle of claim 43 wherein the metal-chelated polyphenol complex nanoparticle is formed by (i) the metal-chelated polyphenol complex, (ii) the particle aggregation inhibiting conjugated lipid and (iii) the non-cationic lipid or the non-ionizable lipid, the molar proportion of the metal-chelated polyphenol complex in starting material is 10% to 20%, the molar proportion of the particle aggregation-inhibiting conjugated lipid in starting material is 2% to 10%, the molar proportion of the cholesterol in starting material is 0% to 48%, and molar proportion of the non-cationic lipid or the non-ionizable lipid other than cholesterol in starting material is 40% to 75%.

55. The metal-chelated polyphenol complex nanoparticle of claim 43, wherein the metal-chelated polyphenol complex nanoparticle is formed by (i) the metal-chelated polyphenol complex, (ii) the particle aggregation-inhibiting conjugated lipid and (iii) the non-cationic lipid or the non-ionizable lipid, the molar proportion of the metal-chelated polyphenol complex in starting material is 5% to less than 10%, the molar proportion of the particle aggregation-inhibiting conjugated lipid in starting material is 2% to 10%, the molar proportion of the cholesterol in starting material is 0% to 48%, and the molar proportion of the non-cationic lipid or the non-ionizable lipid other than cholesterol in starting material is 30% to less than 40% or 40% to 75%; or
the metal-chelated polyphenol complex nanoparticle is formed by (i) the metal-chelated polyphenol complex, (ii) the particle aggregation-inhibiting conjugated lipid and (iii) the non-cationic lipid or non-ionizable lipid, the molar proportion of the metal-chelated polyphenol complex in starting material is 10% to 20%, the molar proportion of the particle aggregation-inhibiting conjugated lipid in starting material is 2% to 10%, the molar proportion of the cholesterol in starting material is 0% to 48%, and molar proportion of the non-cationic lipid or the non-ionizable lipid other than cholesterol in starting material is 30% to less than 40%.

56. The metal-polyphenol composite particle of claim 54 or 55, wherein the molar proportion of the metal-chelated polyphenol complex in starting material is 5% to less than 10%, 10% to 15%, or 15% to 20%.

57. The metal-chelated polyphenol complex nanoparticle of claim 56, wherein the molar proportion of the metal-chelated polyphenol complex in starting material is 5%, 10% or 15%.

58. The metal-chelated polyphenol complex nanoparticle of claim 54 or 55, the molar proportion of the particle aggregation-inhibiting conjugated lipid in starting material is 3-5% or 5-10%.

59. The metal-chelated polyphenol complex nanoparticle of claim 58, the molar proportion of the particle aggregation-inhibiting conjugated lipid in starting material is 3%, 5% or 10%.

60. The metal-polyphenol composite particle of claim 54 or 55 wherein the molar proportion of cholesterol in starting material is 10% to 30%, 30% to 47% or 10% to 20%.

61. The metal-chelated polyphenol complex nanoparticle of claim 60, wherein the molar proportion of cholesterol in starting material is 10%, 30% or 47%.

62. The metal-chelated polyphenol complex nanoparticle of claim 54 or 55, wherein the molar proportion of the non-cationic lipid or the non-ionizable lipid other than cholesterol in starting material is 45% to 55%, 60% to 65% or 50% to 65%.

63. The metal-chelated polyphenol complex nanoparticle of claim 62, wherein the molar proportion of the non-cationic lipid or the non-ionizable lipid other than cholesterol in starting material is 45%, 55%, 60% or 65%.

64. The metal-chelated polyphenol complex nanoparticle of claim 54 or 55, wherein the molar proportion of the metal-chelated polyphenol complex in starting material is 5% to less than 10% or 10% to 15%, the molar proportion of the particle aggregation-inhibiting conjugated lipid in starting material is 5% to 10%, the molar proportion of the cholesterol in starting material is 10% to 30%, and the molar proportion of the non-cationic lipid or the non-ionizable lipid other than cholesterol in starting material is 60% to 65%, and the metal ion moiety in the metal-chelated polyphenol complex is Fe³⁺.

65. The metal-chelated polyphenol complex nanoparticle of claim 64, wherein the molar proportion of the metal-chelated polyphenol complex in starting material is 15%, the molar proportion of the particle aggregation-inhibiting conjugated lipid in starting material is 10%, the molar proportion of the cholesterol in starting material is 10%, and the molar proportion of the non-cationic lipid or the non-ionizable lipid other than cholesterol in starting material is 65%, and the metal ion moiety in the metal-chelated polyphenol complex is Fe³⁺.

66. The metal-chelated polyphenol complex nanoparticle of claim 64, wherein the molar proportion of the metal-chelated polyphenol complex in starting material is 5%, the molar proportion of the particle aggregation-inhibiting conjugated lipid in starting material is 5%, the molar proportion of the cholesterol in starting material is 30%, and the molar proportion of the non-cationic lipid or the non-ionizable lipid other than cholesterol in starting material is 60%, and the metal ion moiety in the metal-chelated polyphenol complex is Fe³⁺.

67. The metal-chelated polyphenol complex nanoparticle of claim 54 or 55, wherein the molar proportion of the metal-chelated polyphenol complex in starting material is 5% to less than 10% or 10%, the molar proportion of the particle aggregation-inhibiting conjugated lipid in starting material is 3% to 5%, the molar proportion of the cholesterol in starting material is 30% to 47%, and the molar proportion of the non-cationic lipid or non-ionizable lipid other than cholesterol in starting material is 45% to 55%, and the metal ion moiety in the metal-chelated polyphenol complex is Al³⁺ .

68. The metal-chelated polyphenol complex nanoparticle of claim 67, wherein the molar proportion of the metal-chelated polyphenol complex in starting material is 5%, the molar proportion of the particle aggregation-inhibiting conjugated lipid in starting material is 3%, the molar proportion of the cholesterol in starting material is 47%, and the molar proportion of the non-cationic lipid or the non-ionizable lipid other than cholesterol in starting material is 45%, and the metal ion moiety in the metal-chelated polyphenol complex is Al³⁺; or
wherein the molar proportion of the metal-chelated polyphenol complex in starting material is 10%, the molar proportion of the particle aggregation-inhibiting conjugated lipid in starting material is 5%, the molar proportion of the cholesterol in starting material is 30%, and the molar proportion of the non-cationic lipid or the non-ionizable lipid other than cholesterol in starting material is 55%, and the metal ion part in the metal-chelated polyphenol complex is Al³⁺.

69. The method of preparing a metal-chelated polyphenol complex nanoparticle of any one of claims 28 to 68, wherein (i) a metal-chelated polyphenol complex, (ii) a particle aggregation-inhibiting conjugated lipid and (iii) a non-cationic lipid or a non-ionizable lipid are mixed to obtain the metal-chelated polyphenol complex nanoparticle.

70. The method of claim 69, wherein the method comprises the steps of:
(1) forming a metal-chelated polyphenol complex by reacting a polyphenol molecular moiety and a metal ion moiety through a coordination bond;
(2) mixing the metal-chelated polyphenol complex prepared in the step (1), a particle aggregation-inhibiting conjugated lipid and a non-cationic lipid or a non-ionizable lipid to obtain the metal-chelated polyphenol complex nanoparticle.

71. The method of claim 70, wherein the metal-chelated polyphenol complex is obtained by dissolving a polyphenol molecule in ethanol and then adding a metal ion.

72. The preparation method of claim 71, wherein the molar ratio of the polyphenol molecules to the metal ions is 1: (1-2).

73. The method of claim 71, wherein the reaction conditions include a reaction temperature of 60 °C and a duration of 1 hour.

74. A drug-lipid particle, wherein the drug-lipid particle comprises:
(a) a drug, the drug is a negatively charged molecule; and
(b) the metal-chelated polyphenol complex nanoparticle of any one of claims 28 to 68.

75. The drug-lipid particle of claim 71, wherein the drug is encapsulated in the metal-chelated polyphenol complex nanoparticle.

76. The drug-lipid particle of claim 75, wherein the drug is selected from the group consisting of a nucleic acid, a protein, a polypeptide, a small molecule, a nucleic acid analog, a protein analog, and a polypeptide analog, and combinations thereof.

77. The drug-lipid particle of claim 76, wherein the nucleic acid is selected from the group consisting of mRNA, siRNA, sgRNA, ASO, circRNA, microRNA, DNA, ecDNA and artificial nucleic acid, and combinations thereof.

78. The drug-lipid particle of claim 77, wherein the nucleic ac id is an mRNA sequence shown in SEQ ID No.1 for encoding eGFP, an mRNA sequence shown in SEQ ID No.2 for encoding a receptor binding domain RBD of a novel coronavirus S1 subunit, an mRNA sequence shown in SEQ ID No.3 for encoding NY-ESO-1, an siRNA sequence of a Bcl-2 gene having an antisense strand shown in SEQ ID No.4 and a sense strand shown in SEQ ID No.21, an siRNA sequence of a PLK1 gene having an antisense strand shown in SEQ ID No.6 and a sense strand showed in SEQ ID No.23, an siRNA sequence of a Gal-1 gene shown in SEQ ID No.8, an ASO sequence of a STAT-3 gene shown in SEQ ID No.10, an ASO sequence of an alpha-syn gene shown in SEQ ID No.12, an ASO sequence of a Bcl-2 gene shown in SEQ ID No.14, an mRNA sequence shown in SEQ ID No.16 for encoding a wild-type novel coronavirus S protein, a double-stranded DNA sequence having an antisense strand shown in SEQ ID No.17 and a sense strand shown in SEQ ID No.25, a single-stranded DNA shown in SEQ ID No.18, or a siRNA sequence of a B7-H4 gene having a sense strand shown in SEQ ID No.19 and an antisense strand shown in SEQ ID No. 26.

79. A preparation method for the drug-lipid particle of any one of claims 74 to 78, wherein the drug is encapsulated in the metal-chelated polyphenol complex nanoparticle to obtain the drug-lipid particle.

80. The preparation method of claim 38, wherein the drug-lipid particle is obtained by mixing (a) a drug, (i) a metal-chelated polyphenol complex, (ii) a particle aggregation-inhibiting conjugated lipid, and (iii) a non-cationic lipid or a non-ionizable lipid.

81. The preparation method of claim 80, wherein the metal-chelated polyphenol complex, the particle aggregation-inhibiting conjugated lipid, and the non-cationic lipid or the non-ionizable lipid are dissolved in an organic compound to form an organic phase, the drug is dissolved in a buffer to form an aqueous phase, and the organic phase and the aqueous phase are mixed to obtain the drug-lipid particle.

82. The preparation method of claim 81, wherein the organic compound is ethanol.

83. The method of claim 81, wherein the buffer is an enzyme-free Tris-HCl buffer.

84. The method of claim 81, wherein mixing means of the organic phase and the water phase includes micro-fluidic chip or ultrasound.

85. Use of the metal-chelated polyphenol complex nanoparticle of any of claims 28 to 68 in a drug-lipid particle.

86. Use of the metal-chelated polyphenol complex nanoparticle of any of claims 28 to 68 or the drug-lipid particle of any of claims 74 to 78 in a composition for the delivery of a drug.

87. The use of claim 86, wherein the composition is used for introducing the drug into a cell.

88. The use of claim 86, wherein the composition is a medicament.

89. The use of claim 88, wherein the medicament is used for silencing expression of a target sequence in a mammalian subject, for delivering a drug in the body of a mammal, for delivering a drug into mammalian cells from the body, or for treating a disease or disorder in a mammal.

90. The use of claim 89, wherein the mammal is a human.

91. The use of claim 89, wherein the disease or disorder is associated with expression of a gene which comprises a target sequence of a drug.

92. The use of claim 89, wherein the disease or disorder comprises cancer, a viral infection, an autoimmune disease, diabetes, and Alzheimer's disease.

93. The use of claim 92, wherein the viral infection comprises hepatitis A, hepatitis B, hepatitis C, SARS-Cov-2, HIV, HPV, influenza, smallpox, and syphilis.

94. The use of claim 92, wherein the cancer comprises liver cancer, glioma, melanoma, lung cancer, pancreatic cancer, and breast cancer.

95. The use of claim 88, wherein the medicament is a vaccine.

96. The use of claim 88, wherein the route of administration of the medicament comprises intrathecal injection, intramuscular injection, intracranial injection, intravenous injection, and intratumoral injection.

97. A medicament containing the metal-chelated polyphenol complex nanoparticle of any of claims 28 to 68 or the drug-lipid particle of any of claims 74 to 78.

98. The medicament of claim 97, wherein the medicament is a vaccine.

99. The medicament of claim 98, wherein the vaccine is a novel coronavirus vaccine.

100. Use of the metal-chelated polyphenol complex nanoparticle of any of claims 28 to 68 or the drug-lipid particle of any of claims 74 to 78 in the prevention and/or treatment of a disease or disorder in a mammal.

101. The use of claim 100, wherein the mammal is a human.

102. The use of claim 100, wherein the disease or disorder is associated with expression of a gene which comprises a target sequence of a drug.

103. The use of claim 100, wherein the disease or disorder comprises cancer, a viral infection, an autoimmune disease, diabetes, and Alzheimer's disease.

104. The use of claim 103, wherein the viral infection comprises hepatitis a, hepatitis b, hepatitis c, SARS-Cov-2, HIV, HPV, influenza, smallpox, and syphilis.

105. The use of claim 103, wherein the cancer comprises liver cancer, glioma, melanoma, lung cancer, pancreatic cancer, and breast cancer.
